# EUROPEAN PATENT APPLICATION

(11) **EP 1 067 181 A1**
(43) Date of publication of application: **10.01.2001**
(21) Application number: 99201846.5
(22) Date of filing: 10.06.1999
(51) Int. Cl.: C12N 15/10, C12N 15/56, C12N 15/62, C12N 15/70, C12N 9/28, C12N 1/21, C12Q 1/68

(54) **Enzyme selection**

(71) Applicant: Rijksuniversiteit te Groningen, 9712 CP Groningen (NL)
(72) Inventor: The designation of the inventor has not yet been filed
(74) Representative: Ottevangers, Sietse Ulbe

(57) **Abstract**

The invention relates to the production by recombinant technology of enzymes having desired activity.

The invention provides methods employing display vehicle technology allowing to select enzymes having desired catalytic activity. The invention provides a method for selecting an enzyme mutant with desired catalytic activity comprising displaying each of a multitude of mutants of said enzyme on the surface of a display vehicle comprising a nucleic acid encoding said mutant and selecting for a display vehicle carrying nucleic acid encoding an enzyme mutant having at least a site other than its catalytic site mutated.

## Description

The invention relates to the production by recombinant technology of enzymes having desired activity.

Many proteins are enzymes: catalysts that accelerate the rate of specific reactions. Enzymes generally have at least two (sometimes overlapping) key regions in their functional sites: one for binding the substrate, another for catalysing the chemical reaction on the bound substrate. An enzyme-substrate complex frequently functions as an intermediate in catalysis, the binding of a substrate to an enzyme may induce a conformational change in the enzyme that positions the catalytic residues correctly so that catalytic action can occur.

Most enzymes do not function at a constant rate because allosteric activators or inhibitors bind at yet other functional sites (often separate from the substrate binding site) to modulate the catalytic activity of enzymes. Molecules that bind to enzymes and increase or decrease their activities are called effectors. Effectors can modify enzymatic activity because enzymes can assume both active and inactive conformations; activators are positive effectors, inhibitors are negative effectors. Effectors bind at regulatory sites or allosteric sites, a term used to emphasise that the regulatory site is an element of the enzyme distinct from the catalytic site and to differentiate this form of regulation from competition between substrates and inhibitors at the catalytic site.

Furthermore, an enzyme that catalyses one or more of a series reactions is often inhibited by a product from the reactions series, this process is called feedback inhibition. A multimeric enzyme can have multiple substrate binding sites; the binding of a substrate at one site can cause a conformational change that modifies the affinity of the other site(s), making the enzyme responsive to a narrow range of substrate concentrations. Other functional sites include for example product inhibition sites.

The conditions (herein called environmental or physiological conditions) under which enzymatic catalysis optimally occurs are often narrowly defined, and depend among others on the presence or concentration of electrolyte, salt, substrate or product thereof, on the presence of competing substrate or inhibitor, on the presence of heterologous enzyme (such as a protease effectively hydrolysing the enzyme in question, or an enzyme having different catalytic activity but competing for the same substrate), on the presence of an effector, or on other conditions such as temperature, and on combinations of these conditions. In general, extreme conditions, such as high salt, high temperature, or high or low pH, diminish, or at least modulate, the desired catalytic activity.

Many enzymes find use as catalysts that accelerate the rate of specific reactions in industry. Enzymes find their use in processing of food, in laundry soaps, in production of fine (bio)chemicals, to name a few. Often, in industrial use, environmental conditions for the process are required or sought that deviate from those that are optimal for the catalytic activity for the enzyme in question. For example, enzymatic protease in laundry soaps is often required to function under conditions of high salt and temperature, optionally after the addition of bleach, thereby seriously effecting its proteolytic activity.

Several protein engineering or genetic modification techniques are now at hand. Quite promising is the phage display technique, where a bacteriophage is used as a display vehicle, that is a vehicle carrying a nucleic acid encoding the protein or enzyme in question, whereby said protein is displayed in bound form at the surface of the display vehicle. Other display vehicles comprise for example bacteria or other viruses. A library of vehicles, all carrying diverse genetic information in said nucleic acid and displaying proteins can be used as source to select a protein binding to a desired substance. Well known are antibody displaying phages, that are used to select phages containing nucleic acid encoding a desired (synthetic) antibody. Binding of display vehicles through their respective displayed protein to a desired substance allows selection of display vehicles carrying relevant nucleic acid relating to binding. In general, selection of such vehicles is a multistep process. A round of selection of appropriate vehicles by testing for binding and obtaining those that preferentially bind is followed by amplification of those selected vehicles. Selected pools of amplified vehicles, being enriched in desired nucleic acid, are than used in a subsequent selection round. Amplification of a bacteriophage or other virus occurs in an appropriate cell culture, bacteria can be amplified in a bacterial culture. The desired nucleic acid of thus selected display vehicles can of course after selection and/or amplification be genetically modified by standard DNA recombinant technology.

The present invention provides methods employing display vehicle technology allowing to select enzymes having desired catalytic activity. In a first embodiment, the invention provides a method for selecting an enzyme mutant with desired catalytic activity comprising displaying each of a multitude of mutants of said enzyme on the surface of a (corresponding) display vehicle comprising said nucleic acid encoding said mutant and selecting for a display vehicle carrying nucleic acid encoding an enzyme mutant having at least a site other than its catalytic site mutated. The invention herewith deviates from common current practice, where one in general, focuses at modifying catalytic sites of an enzyme. To modify catalytic activity, recombinant technology, using for example site-directed mutation techniques, is finding more and more use in modifying catalytic sites of enzymes and selecting or producing one with the desired catalytic activity under the required conditions. However, genetically modifying amino acid residues at catalytic sites most times does not result in an enzyme mutant having maintained its desired catalytic activity, most of the times catalytic activity is abolished altogether, and finding the proper modification is often the result of much experimental trial and error. Paradoxically, the invention provides modification of at least one other functional site than a catalytic site, in order to achieve modified catalytic activity.

Preferably, said mutated site comprises a substrate binding site or allosteric site of said enzyme, whereby the mutated enzyme is changed (most likely in its conformational characteristics) to allow for better catalytic activity. The selection of a desired enzyme-displaying display vehicle, e.g. by binding it to a substrate or effector, leads to the selection and isolation of the nucleic acid that codes for the mutant enzyme. For example, the invention provides a method wherein one selects on the characteristics of a substrate binding site or nucleic acid encoding therefore, for example by selecting for improved binding of said enzyme displayed on said surface with a substrate for said enzyme. Herewith, the binding of the substrate to a mutated binding site of the enzyme induces for example an improved conformation in the enzyme that positions the catalytic residues correctly so that catalytic action is improved. In another example, the invention provides a method wherein one selects on the characteristics of a regulatory or allosteric site or nucleic acid encoding therefore, for example by selecting for improved binding of said enzyme displayed on said surface with an effector for said enzyme. Herewith, a mutant enzyme is obtained wherein enzymatic activity is modified because effectors, such as activators or inhibitors, have different and improved effects on the enzyme's conformation, allowing improved or altered catalytic activity upon interaction with said effector.

In a preferred embodiment, the invention provides a method wherein said binding to said site such as a substrate binding or regulatory site occurs under a set environmental condition. With the phrase "a set environmental condition", the conditions are meant that are used in the onset of the experimental tests to test for improved binding. A person skilled in the art knows how to vary experimental conditions such as the presence or concentration of electrolyte, salt, substrate or product thereof, the presence of competing substrate or inhibitor, the presence of heterologous enzyme (such as a protease effectively hydrolysing the enzyme in question, or an enzyme having different catalytic activity but competing for the same substrate), the presence of an effector, or other experimental conditions such as temperature, and combinations of these conditions dependent on the aim for or purpose of the mutant searched for. By measuring various of these parameters during the experiment, it is often even possible to maintain these conditions, or stay at least close to them, during the experiment as they are set at the onset of the experiment. By selecting desired environmental conditions, it is possible to select for enzymes having desired catalytic activity under those conditions. As an example, the detailed description herein describes how to obtain a mutant enzyme having desired catalytic activity at lower pH, said mutant enzyme having improved catalytic activity at lower pH than the corresponding wild-type enzyme.

Another embodiment provides a method according to the invention wherein said binding of said enzyme displayed on said surface is tested in at least two rounds of selection. Repeated binding and elution of the display vehicle allows for better selection of the desired mutant enzyme. Preferably, a method is used wherein at least between two rounds of selection said display vehicle is not amplified. Contrary to the common grain of wisdom, where amplification of display vehicles between selection rounds is common place, in order to enrich the pool of desired gene vehicles genetically, the invention provides a method for selecting a mutant protein, such as an enzyme but not necessarily restricted thereto, or mutant part thereof comprising displaying each of a multitude of mutants of said protein or part thereof on the surface of a corresponding display vehicle comprising a nucleic acid encoding said mutant and selecting for a display vehicle carrying nucleic acid encoding a mutant by successive rounds of selection wherein at least between two successive rounds of selection said display vehicle is not amplified. Such a repetitive selection method (herein also called quick select) provides a much stronger and adjustable method because it allows subjecting a selected pool of display delivery particles to another selection round without the need of amplification. The vehicles that are eluted during a first round of selection are used in a second one without intermediate amplification. If so desired, set environmental conditions can be changed in between rounds as well. This procedure improves the selection procedure even more. Amplification as used commonly selects for mutants that have lost their insert by outgrowing those that have retained the desired protein sequence. The invention therefore provides to omit at least one of the growing/amplification rounds in the procedure commonly used; by starting from a large display vehicle pool the invention provides to isolate a proper binder by two or more successive rounds of binding and elution, without amplification in between.

The invention also provides herewith a mutant protein (such as a mutant enzyme) or mutant part thereof selectable and hence obtainable using quick select, and a nucleic acid encoding said mutant protein or part thereof. Also the invention provides a vector, such as a plasmid, cosmid or YAC vector, or viral vector, such as a phage, comprising said nucleic acid as provided by the invention. Furthermore, the invention provides a host cell, such as a prokaryotic (bacterial) cell or eukaryotic (yeast, plant or mammalian) cell comprising a nucleic acid or vector, or at least a functional part therof, according to the invention. For example, the invention provides a mutant enzyme selectable according the invention, such as an enzyme with altered substrate binding properties, for example an enzyme which has a separate binding and catalytic domain, comprising altered substrate or effector binding properties. In particular under altered or adapted physiological or environmental conditions, such as altered pH, altered salt, altered temperature, presence of proteases and so on.

In a particular embodiment, the invention provides a mutant enzyme wherein its catalytic site is at least partly distinct from its substrate binding site or its allosteric site, such as transferases, ligases, etc., or such as for example a polysaccharide binding enzyme having improved catalytic activity over its wild-type enzyme at a set environmental condition. Examples of such enzymes are CGTase (cyclomaltodextrin glycosyltransferase EC 2.4.1.19) and other glucosyltranferases (EC 2.4.1), cellulase (EC 3.2.1.4), xylanase (EC 3.2.1.8), chitinase (EC 3.2.1.14) and other carbohydrate hydrolysing enzymes (EC3.2.1.), pectin lyase (EC4.2.2.10), xanthane lyase. (EC 4.2.2.12) and other glucanlyases (EC 4.2.2.). In the detailed description a mutant α-amylase is provided. Such a mutant enzyme can for example be obtained from any well known industrial microorganism, such as Sacharomyces, Aspergillus, Lactobacillus, Lactococcus or Bacillus. For example, the invention provides an enzyme derived from *Bacillis licheniformis* having improved catalytic activity over its corresponding wild-type enzyme at pH 4.5. The invention furthermore provides a nucleic acid encoding a mutant enzyme according the invention, a vector comprising said nucleic acid and a prokaryotic or eukaryotic host cell comprising said nucleic acid or vector according to the invention. The invention also provides a method to obtain a desired product comprising altering a substrate by subjecting said substrate to the catalytic activity of an enzyme according to the invention. For example, the invention provides such a method wherein said substrate comprises a polysaccharide. Also, the invention provides a product obtainable by a method according to the invention for example using an enzyme obtainable using a method according to the invention. In the detailed description an example is given relating to variants of a parent B. licheniformis α-amylase mutated in the C-terminal domain having an improved performance at a pH lower than 6, 5.9, 5.5, 5, or even 4.5. α-Amylase can be used for the liquefaction of starch. The invention provides to change the starch binding properties of the enzyme using phage display. The use of phage display is provided to select enzymes under altered or adapted physiological conditions: altered pH, altered temperature, altered salt conditions, and so on.

The invention provides a selection system which is based on the ability of α-amylase displaying phages to bind to cross-linked and raw starch. This system was used for example at various pH values. A library of α-amylase mutants that have one or more amino acid residues changed in for example their CB4 starch binding domain was subjected to phage display selection. Mutant molecules with good starch binding and hydrolytic capacity were isolated from the phage library by repeated binding and elution of the α-amylase displaying phage particles at lowered pH value. Also wild type α-amylase was selected. All selected variants could hydrolyse both starch and heptamaltose as well as the wild type α-amylase. Variants were found with a starch hydrolysis ratio at pH=4.5 / pH=7.5 that is improved relative to the wild type α-amylase. The present invention for example provides new amylase variants that have improved starch hydrolysis activity at lowered pH value, provides DNA constructs encoding the variants, and provides vectors harbouring the constructs. For a person skilled in the art it is obvious that the same approach can also be applied to other proteins having a separate substrate binding or effector site and catalytic site. The invention is further explained in the detailed description without limiting the invention.

### Detailed description

α-Amylase (1,4 α-D-glucanhydrolase, EC 3.2.11) catalyses the hydrolysis of α-1,4 glucan linkages to produce maltose and larger oligosaccharides. α-Amylase is used in the large scale liquefaction of corn starch. The enzyme is produced by a variety of microorganisms like Bacillus and Aspergillus. In industry mostly the enzymes from Bacillus (B. *licheniformis, B. stearothermophilus* and B. *amyloliquefaciens)* are used. The enzyme originating from *Bacillus licheniformis* is most thermostable and therefore it is preferred. The process of starch conversion to dextrose typically consists of three steps: gelatinization of the starch, liquefaction of the starch yielding oligosaccharides (polymerisation degree of 7-10), followed by saccharification of the liquefied starch into dextrose.

In the most common process, the starch is mixed as a slurry with α-amylase. For this process it is necessary to change environmental conditions. First, the pH of the slurry has to be adjusted to a value between 5.8 and 6.5, which is in general considered the lower limit for α-amylase. After mixing, the temperature of the slurry is than rapidly increased to between 80 and 115 °C leading to concomitant gelatinization and liquefaction. After this, the mixture is kept at a temperature between 80 and 100 °C for up to three hours to bring down the average chain length to between seven and twelve units. In another process the starch-α-amylase mixture is first incubated at a temperature between 80 and 100 °C to combine gelatinization and hydrolysis, followed by an increase in the temperature to over 105 °C to complete the gelatinization and, after cooling the mixture, an additional supplement of α-amylase to complete the hydrolysis.

None of the processes can be operated at environmemtal conditions comprising a value below pH of 5.9 since the α-amylase does not perform well below that value. (Crabb *et al.,* 1997) Thus the pH of the starch slurry has to be adjusted from 4.5, the natural value, to 5.8-6.5 (Crabb *et al.*, 1997). However, the next step in the conversion to dextrose is the saccharification. This process is necessarily carried out at a pH of 4.2 (preferably between 4.2 and 4.5) and 60 °C, since these are the conditions preferred by the exo 1-4 glycanohydrolase that removes glucose units one by one from the non-reducing end of the oligosaccharide. It is clear that the availability of an α-amylase which would have a good performance at the environmental conditions of the sacchaification, such as low pH, would eliminate the cost now made for the pH adjustments and allow for the co-incubation of the α-amylase with the saccharifying enzyme.

The structure of *B.licheniformis* α-amylase has been resolved and the coordinates have been released recently (Machius *et al.,* 1995, Hwang *et al.,* 1996). To the C-terminal domain of the licheniformis no function has been ascribed (Machius *et al.,* 1995).The α-amylase shows homology with other α-amylases and with the well studied cyclodextrin transferases. However, in the latter structures the C-terminal domain is significantly extended and in the additional domains residues have been identified that are responsible for the starch interaction (Penninga *et al.,* 1996).

Despite the fact that no starch binding domain has been assigned, the enzyme can scroll along the starch surface to break down the complete polymer into oligosaccharides (Helbert *et al.,* 1996). Thus some specific interaction between the enzyme and its complex starch substrate may form the start for the hydrolytic reaction. In other polysaccharide degrading enzymes the catalytic and substrate binding domains are separated, implying that the starch binding property of the protein is independent from the catalytic action of the enzyme. Therefore we decided to use the hereto not studied possible interaction between the C-terminal domain of the B. *licheniformis* α-amylase and the starch substrate to develop an selection method for an α-amylase mutant which has an altered starch binding, but which is still fully functional otherwise. The starch binding property was, without relating this property to specific parts of the protein molecule, already used to develop a large scale purification procedure which involves affinity chromatography to raw starch or cross-linked starch (Weber *et al.,* 1976; Rozie *et al.,* 1990; Satoh *et al.,* 1993; Somers *et al.,* 1995).

We now show that the application of wild-type α-amylase at pH values lower than six is hampered by a decreased starch binding capacity. In the past, mutants have been generated and screened for their halo formation capacity on starch plates. The complete gene was subjected to random mutagenesis and the variants with altered halo formation capacity were selected. None of the mutations selected in those experiments reside in the C-terminal domain (USP 5,736,499, USP 5,364,782). We decided to address specifically the C-terminal domain.

In order to study a relative large sequence space, a technique that selects for enzyme properties, i.e. a technique in which all mutants do not have to be screened separately, is advantageous. The phage display technique offers such a possibility (ref: W. Quax, Phage-enzymes: chances and limits, IBC's third Annual International Conference on Display Technology. Held June 11-12, 1998, The Clift House, San Francisco, USA). The technique was originally developed by Smith (Smith, 1985), who showed that small peptides can be expressed on the surface of a display vehicle such as the filamentous phage fd. A small peptide was expressed as a fusion to the N-terminus of g3p coat protein resulting in the "display" of that peptide. It was shown that it is possible to select for a phage encoding a specific peptide by affinity binding. This principle has been exploited to isolate specific peptides from large libraries of phages, which differ in the peptide they display on their surface. The selection of a desired peptide, e.g. by binding it to an antibody, automatically leads to the co-selection and isolation of the DNA that codes for the peptide. It has been reported that a single phage particle can be selected out of a population of 10⁷ by multiple rounds of selection and enrichment (For a review see Cortese *et al.,* 1994).

Initially the application of this technique was restricted to small peptides. Later it was found that protein domains (Roberts *et al.,* 1992) and even complete proteins can be functionally expressed as fusion proteins to g3p. Also β-lactamase was displayed on the surface of phage fd as a fusion with g3p coat protein. A suicide inhibitor was used to selectively enrich a mutant enzyme (Soumillion *et al.*, 1994). Recently we have demonstrated that also penicillin G acylase, an enzyme requiring post-translational processing in the periplasm, can be expressed actively on the surface of a phage particle.

So by displaying α-amylase on the surface of a phage and by setting up a selection system that discriminates between α-amylases (linked to phages) on their starch binding ability, we here demonstrate that it is possible isolate improved amylases. To the person skilled in the art it is obvious that this method is not necessarily restricted to α-amylase. Many different enzymes with improved substrate binding properties can be selected in a similar fashion.

### EXPERIMENTAL

### Materials

The following bacterial strains were used: *E.coli* GM-1 (Miller et al., 1977), *E.coli* TG1 : Sup E, K 12 Δ (lac-pro), thi, hsd D5/F', traD 36, pro AB, lacIq, lac Z Δ M15 and *E.coli* HB 2151 : K12 Δ (lac-pro), ara, nal^{r} ,thi/F', pro AB, lacIq ,lac Z Δ M15. Bacteriophage M13KO7 and phagemid pCANTAB5E were bought from Pharmacia, phagemid pMc/NdeI was used as decribed (Stanssens *et al.,* 1989). Purified α-amylase from *Bacillus licheniformis* was kindly provided by Gist-brocades N.V. (Delft, The Netherlands). Raw corn starch granules were obtained from AJB, Zaandam, The Netherlands (Trade name: Maizena).

All primers were synthesised by an in-house facility of the Leiden University. The nucleotide sequence coding for a restriction site is given in lowercase typing.

### Methods

### 1. Design and construction of plasmids for α-amylase display and selection

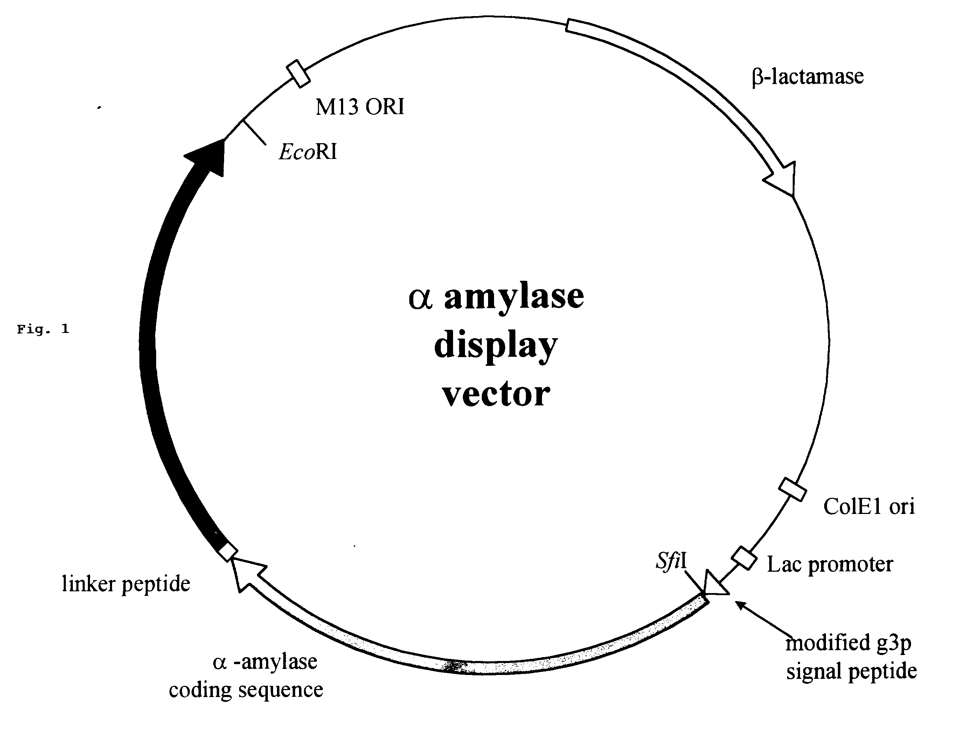

The *B. licheniformis* α-amylase gene from plasmid pLAT3 (WO85/00382) was amplified using oligonucleotides Af1 (5'-C TTC TTG CTG CCT CAg gcc cag ccg gcc ATG GCA AAT CTT AAT GGG-3') and Arl (5'-CC GTC CTC TCT Ggc ggc cgc TCT TTG AAC ATA AAT TG-3'). The amplified DNA fragment was digested with *Sfi*I and *Not*I, and cloned into vector pCANTAB5E. This resulted in plasmid pRa1. The lacZ inducible sequence now codes for an α-amylase fused to the g3p coat protein of bacteriophage fd. Using *Hind*III/*Not*I digestion the α-amylase coding sequence was inserted in expression/display vectors which have a his tag interlaced between the α-amylase and the g3p, which result in display on g8p or which have the amber stop codon between the α-amylase and the phage coat protein removed (Fig. 1).

### 2. Phage rescue, titer determination, phage electrophoresis and western blotting

Phage particles were rescued similar to the procedure described earlier. After infection with M13KO7 phage, cells were spun down at 2500xg for 5 minutes, and resuspended in 100 ml 2xYT medium with ampicillin and kanamycin. Cultures were shaken (250 rpm) overnight at 28°C. From the supernatant of the overnight culture, phagemids were precipitated with 4% PEG6000/0.5M NaCl. Pellets were resuspended in 1 ml 10 mM Hepes pH=7.

To determine the titer routinely, phagemid solutions were diluted in 1 ml *E. coli* GM-1 (Miller *et al.,* 1977) cell suspension (A650=0.4). After selection rounds, very low titer values in eluens were determined by the dilution of 25-50 µl of eluent with ten volumes of cells. This procedure was developed because a high concentration of adsorption buffer inhibits infection. The mixture was left at room temperature for 15 minutes, and plated on antibiotic-containing plates. Phagemid samples were separated by overnight electrophoresis in 2% agarose, buffered by 25 mM Tris/250 mM glycine, pH = 8.6 at 20 V as described earlier for penicillin G acylase displaying phage particles. Part of the gel was briefly soaked in a 0.5% SDS solution, and blotted onto nitrocellulose (Schleicher & Schuell). α-Amylase on the blot was detected by polyclonal antiserum against α-amylase from *B. licheniformis* raised in rabbits and alkaline phosphatase-linked anti-rabbit goat antibodies (Sigma). A comparable part of the gel, loaded with the same samples was stained by ethidium bromide in a 0.1N NaOH solution and destained in water, to detect DNA.

### 3. Identification of amino acid residues for randomisation, construction of the phage library and analysis for its diversity.

The residues for randomisation were selected by visual inspection of the 3D structure of the *licheniformis* enzyme as present in the Brookhaven Protein Data bank (1bpl), using the Insight program (Biosym). The sequences selected were also analysed for homology with the amino acid sequence of other α-amylases and glucosidases.

The library was constructed as follows: A spiked oligo (Bio744: 5'-CATTAATAACAGACGGACCCGGTGGGGCAAAGCGAATGTATGTCGGCCGGCAAAAC-3') was prepared by 25% randomisation of each nucleotide in de coding sequence for residues 433 to 439 (underlined sequence). Then two fragments was generated: One using the primers set Bio619 (5'-GG CAA TCG CTT GAg tcg acT GTC-3' *Hinc*II) and Bio743 5'-GTCCGTCTGTTATTAATGCC-3'); the other with Bio744 and Bio561 5'-C GAA Tag atc tTC ATT AAA GCC AG-3') They were combined by overlapping PCR (Expand™ High Fidelity System (Boehringer) using primers Bio561 and Bio619. The pool of phagemids was constructed by the insertion of this DNA after *Sal*I/*Pst*I digestion into the *Sal*I/*Pst*I sites of plasmid pRα-His-Amb-g3p. The library was generated by transformation of PEG/DMSO processed cells. Phages were rescued as described above using 250 µl library cells as the inoculum, 100 ml LB with 2% glucose and 0.1 µg/ml Amp as growth medium until the OD became 0.4 and M13KO7 (2x10¹¹ colony forming units) were added. After incubation (1 hr) cells were collected and resuspended in 100 ml 2xYT medium containing kanamycin and ampicillin and grown overnight (28°C) Cells were removed and phagemids precipitated with PEG/NaCl. Phage particles were resuspended in 5 ml 10 mM Tris-Cl pH7.0/ 1mm EDTA and filtered through a 0.45 µm filter to remove residual cells.

The diversity of the library was checked by dideoxy sequencing of individual colonies. From a set of 32 mutants 19% had one amino acid change, 25% had two changes, 25% had three, 13% had four, 16% five, <3% six, <3% seven. These values are close to the theoretical percentages of 15, 28, 28, 17, 4, 0.5, 0.1 respectively. Wild-type nucleotide sequences were present in 10% of the cases, while this number should be ^{~}3.5% based on the design of the spiked oligonucleotide. This effect may result from incomplete digestion and religation of the recipient vector pRα-His-Amb-g3p.

The integrity of the different constructs was also screened using the possibility of α-amylase producing cells to form halos on either LBZ (LB medium with 1.5% bactoagar and 1% starch) or MMZ plates (M9 salts: 12.8 g/l Na₂HPO₄.7 H₂O, 3 g/l KH₂PO₄, 0.5 g/l NaCl, 1.0 g/l NH₄Cl; 50 µg/l thiamine, 50 mg/1 MgSO₄, 1.5% bactoagar, 1% starch). A similar library can be prepared from other regions of a substrate binding site, such as identified by the residues 402YFDHH406, 446GETWHDITGN455, 456RSEPVVINSE465, 465EGWGEFHVN473, in said α-amylase.

### 4. Selection procedures: Affinity chromatography and Quick select

Starch (25 mg) was suspended in 0.5 ml 100 mM sodium acetate (NaAc) buffer (pH=4.5-6.0). The starch was pelleted (1 min at 14,000xg) and te supernatant removed. Then the material was preincubated with 0.5 ml adsorption buffer (100 mM NaAc pH=4.5-6.0, 1% ovalbumin + 0.2% Tween 20 + 20% glycerol) on ice for 15-20 minutes. Subsequently, either enzyme or phagemids were added in a 0.1 ml volume and left on ice for 20 minutes. Non-adsorbed enzyme or phages were removed by brief centrifugation (1 min.at14,000xg), after which the starch material was washed three times with ice cold adsorption buffer. Finally, bound enzyme or phages were eluted by incubation at 70°C for 5 minutes in 0.5 ml adsorption buffer at pH 5-6.0. Starch material was removed by centrifugation. Eluent was collected and a sample was taken for titer and composition analysis.
For an additional round of selection the phage suspension was not amplified but used directly on a new, freshly prepared affinity material (quick select). The suspension was adjusted to the desired pH by the addition of 5-25 µl 3M NaAc pH=4.7.

### 5. Analysis of mutants: Halo formation and preparation of the periplasmic proteins

On plate the presence of an intact α-amylase copy was determined using starch (Sigma) in either LBZ (LB medium with 1.5% bactoagar and 1% starch) or MMZ plates (M9 salts: 12.8 g/l Na₂HPO₄.7 H₂O, 3 g/1 KH₂PO₄, 0.5 g/l NaCl, 1.0 g/l NH₄Cl; 50 µg/l thiamine, 50 mg/l MgSO₄, 1.5% bactoagar, 1% starch).

Plasmid DNA from isolated mutants selected in *E. coli* suppressor cells (TG1) was transformed to HB2151 cells and selected for their growth on ampicillin and nalidixic acid. A colony was picked and grown overnight. From part of this culture the DNA was isolated and checked by restriction analysis. 200 ul of the same culture was used to inoculate 10 ml of 2xYT, 0.1% glucose, ampicillin medium and grown for 3 hr at 28 C at 300 rpm. Then 5 ml of the same medium with an additional 3 mM IPTG was added and the cells were grown overnight (25 C, 250 rpm). The cells were pelleted and periplasmic proteins were liberated by incubating the cells on ice for 10 minutes in 500 ul PBS containing 1 mM EDTA. Cells were removed by centrifugation (10'at 14,000xg, 4C). The supernatant containing the desired periplasmic proteins was collected and stored frozen.

### 6. Enzyme assays: Activity on EPS, modified starch and soluble starch

The activity of the enzyme on the small nitrophenyl releasing substrate EPS (4-nitrophenyl-α-D-maltoheptaoside-4,6-O-ethylidene from Boehringer) was carried out following the protocol supplied by the supplier. This continuous assay determines the α-amylase catalysed hydrolysis of a maltoheptasoide into oligosaccharides. The presence of α-glucosidase liberates the nitrophenyl moiety.

Hydrolysis of starch was routinely measured by the degradation of modified starch (Phadebas, Pharmacia Sweden). In that assay the hydrolysis of the crosslinked starch liberates a blue dye, allowing the determination of enzyme activity on its natural substrate. This activity is related to reference activities presented in by the datasheet from the supplier. Both assays were carried out at pH=7.5. The wildtype enzyme and the mutants (50 µl) were incubated with an equal volume of substrate solution at 40 C for 30 minutes. The reaction was stopped with 10 µl 0.5 M HCl. The amount of residual starch was stained by 1 ml color solution and the absorption at 620 was measured promptly. The composition of the substrate solution was as follows: 2% Starch (Merck 101257), 9.6 mM Hepes/9.6 mM MES/9.6 mM Glycine buffer (HMG buffer) of pH=4.5 or pH=7.5, 20 mM CaCl₂. The starch was taken from a 4% stock solution. This stock was prepared by supending 5 g starch in 20 ml of water followed by dissolving the starch by the addition of 75 ml of boiling water. The final was adjusted to 125 ml after cooling of the solution. The color solution was prepared by diluting a standard iodine solution (Fluka) five times.

### EXAMPLES

### Example 1: Display of α-amylase of B. licheniformis on phage fd

The α-amylase from of α-amylase gene of *B. licheniformis* was isolated from the plasmid pLat3 (ref) using the primers Af1 and Ar1 and incorporated in the pCANTAB5E plasmid yielding a g3p fusion (pRα1, Fig. 1). The peptide sequence at which the signal peptidase has to cut was derived from the original sequence of the α-amylase and the successful sequence from the penicillin G acylase display vector. *E. coli* non amber suppressor cells were transformed with the construct and grown. The construct produced active α-amylase in the periplasm of these *E.coli* cells.

Subsequently phagemids of the construct were rescued in E. coli suppressor cells. Electrophoretically separated phages were immunostained with rabbit anti-α-amylase-antibodies, or with ethidium bromide to visualize the DNA. Indeed, the α-amylase comigrates with the DNA. This strongly suggests that the α-amylase is covalently linked to the phagemid.
In order to facilitate handling of the α-amylase the gene was transferred to another display vector (pRα-His-Amb-g3p). In this plasmid a collagenase-sensitive site (GPGGP) and a His-tag (HHHHHH) are located between the α-amylase and the g3p. Immunostaining of the enzyme and the DNA indicated that also phages rescued from these cells display the α-amylase on their surface.

We calculated the enzymatic properties of the phagemid particles by measuring the activity per milligram of phagemid protein. These values were converted into a turnover rate (mol of product formed per mol phagemid per second) by estimating the molecular weight of phagemids (2.5x10⁹ mg/mol, {Model & Russel, 1988}. The turnover number of the phagemids was 8.7 s⁻¹. By comparing the turnover rate of the phagemids with that of the free enzyme (185 s⁻¹), we calculated that approximately 5 % of the phage particles carry a copy of the enzyme linked *via* their g3p coat protein.

### Example 2: Affinity selection of α-amylase and phagemids carrying α-amylase

Amylase can be purified by affinity chromatography. This procedure involves the binding of the enzyme to raw starch or cross-linked starch. Typically the enzyme is bound to the starch at 0°C and it is eluted by a temperature increase to 70°C. A similar protocol seems appropriate to selectively bind α-amylase coupled to phagemids.

In our experiment we incubated comparable activities of free α-amylase and pRα-His-Amb-g3p phagemid α-amylase with cross-linked starch at 0 °C for 20 minutes. After this period most of the activity was cleared from the supernatant. Subsequent incubation of the starch at 70 °C released all bound activity in the case of the free enzyme, and half of the phage-enzyme related activity. Thus both free enzyme as well as phagemid-linked enzyme could be immobilized by this method and eluted by temperature elevation.

The strength of the α-amylase binding was studied by varying the number washing steps and measuring the amount of enzyme that remained immobilized on the starch. The free enzyme bound very tightly to the starch: Only 10% of the activity was washed off by the first wash, and 3% by a third wash. Typically 50% of the enzyme activity was recovered following a complete adsorption-elution procedure including three rinsing steps.

The binding of the phage enzymes to the starch is expected to be weaker than that of the free enzyme since the α-amylase contains a large proteinaceous load. However, in an experiment following the same protocol as the one for the free enzyme we were still able to recover 6% of the activity. Thus these data suggest that the binding of the phage-displayed α-amylase is sufficiently strong to allow selection out of a pool of non-binding phages.

We tested this by a competition experiment between α-amylase phages and control phages. The α-amylase phages were derived from the pα-His-g3p phagemids and the control phages were pMc phagemids (Stanssens *et al.,* 1989). The number of different phage particles can be determined since the α-amylase phagemids will confer ampicillin resistance to the infected cells, while the pMc infected cells will become chloramphenicol resistant. A mixture of both phagemids was incubated with cross-linked starch, washed three times and eluted. The eluted material was re-adsorbed to a second batch of starch material, washed three times and eluted. The titer of pα-His-g3p phagemids and pMc non-specific phagemids was determined from samples of each step of the procedure. The selectivity of the starch material (being either cross-linked starch or raw starch) is apparent from the ratio of pα-His-g3p to pMc. The ratio increases with a factor of five from 20 (initially) to 100.

Thus the selection of pα-His-g3p over pMc is due to the presence of α-amylase on the surface of the phagemid since both phagemids are rescued by the same helper phage and therefore consist of identical proteins, except for the α-amylase. These data are also supported by other competition experiments between control phages and phages which had less α-amylase displayed or had their α-amylase coupled to g8p or g6p.

### Example 3: Optimization of the phage enzyme selection procedure

A five fold enrichment may not suffice for a potent selection procedure. Therefore we further elaborated on the principle of repeated rounds of selection with intermediate amplification. We compared the control and α-amylase displaying phages. After four selection/amplification rounds the ratio between α-amylase displaying and control phagemids had not changed significantly, indicating that no "good binder" was being selected. Colonies from the fourth round were tested for α-amylase activity and indicated that only 4% of the mutant population was active. DNA sequence analysis on the total population after each round revealed that a deletion mutant had taken over the α-amylase population in the third and fourth amplification rounds. Restriction digestion showed that already in the second round population 50% of the phagemids contained a deletion. This result indicates that the amplification selects for mutants that have lost their insert by outgrowing those that retain the complete α-amylase-g3p sequence. The rapid loss of almost the entire α-amylase insert made us decide to omit the growing/amplification rounds in the procedure. Instead, by starting from a large phagemid pool (4x10¹²) we set out to isolate a proper binder only by successive rounds of binding and elution. Such a repetitive selection method (quick select) offers us a much stronger and adjustable method because we can subject a selected pool of phage particles to another selection round without the need of amplification.

Thus the phages that were eluted during the first round of selection were used in a second one without intermediate amplification. This procedure improves the selection procedure indeed, since the introduction of a second round of selection increases the ratio from 20 (initially) to 70.000 in the second elution. Thus the α-amylase phages have been enriched by a factor 3500. This observation is confirmed by α-amylase enzyme activity measurements (Table 2).
The limits of the quick select method were tested in a pilot experiment in which 10¹⁰ phage particles were used at the start of the selection protocol. Is was possible to lead the phages through up to seven rounds of binding and elution. After those seven rounds approximately 10³ had survived the complete protocol.

We also checked whether the selection was restricted to starch. Besides cross-linked starch only raw starch granules could be used. Non starch solids like cellulose were not applicable to obtain significant enrichment of α-amylase phages over control phages.

The binding of α-amylase phage particles to raw starch granules depends on the pH value. The enrichment decreased a factor of two hundred when the protocol was carried out at pH=4.5. This decrease is not caused by a loss of viability of the incubated of the phages at that pH (Table 2) but by the combination of the increased temperature *and* the lowered pH during phage elution: Less than 1% of the phages survives the five minutes temperature at 70 C *and* pH=4.5.

### Example 4: α-Amylase phagemid library

The C terminal domain of α-amylase is known to be involved in the binding of the protein with starch. In particular, the β-strand of the CB4 domain is the location for close contacts between the glucose residues and the protein. We decided to address the residues that are in CB4 (433GGAKRMY439) for mutagenesis and selection. We used PCR with primers that are partially randomised for those residues. Transformation yielded a library of bacteria (5x10⁴).

Based on the design of the primers we expect that the wild type α-amylase will be present on 3.4% of the phages, a single mutant amino acid on 15 % of the particles, two altered CB4 amino acids on 28% of the mutants, while the other phages will have α-amylase with more amino acid molecules changed. These numbers and the size of the library indicate that our phagemid pool contains all single mutants and'most double mutants in this region (Table 3).

Each specific single amino acid mutant will be present in a very small amount since in all seven positions, each of the 20 amino acids can be incorporated. Due to this large number of possibilities the chance of finding e.g. Phe at position 439 is 0.1%. Each double mutant will occur on 0.003% of the phages. Sequencing 50 random clones confirmed the diversity of the library according to its design (Table 3).

### Example 5: Selection of starch binding domain variants of α-amylase

Selection rounds were carried out independently at 3 different pH values. As expected, the number of rounds of selection that could be carried out decreased as the pH value during the selection was lowered. At pH=6.0 seven rounds of binding-desorption could be carried out, at 4.5 only four. During and after each selection experiment a number of phagemids was picked and analysed.

The selection procedure yields both mutant and wild type α-amylase phages, irrespective of the pH of the selection (Table 4). The selected wildtype α-amylase phagemids can be divided in two groups: The phenotypic wild type α-amylase with wild type genotype and the phenotypic wild type α-amylase with a mutant genotype. One of the latter had even two silent mutations. The selection of such variants indicates that the wild type is strongly selected for and that, despite the limited occurrence of such a nucleotide sequence, these variants can be isolated. Thus we conclude that the selection procedure is sufficiently strong and the wild type phenotype is difficult to improve with respect to its starch binding property. We therefore expect that the (phenotypic) mutant α-amylase phagemids that are selected side by side with the wild type have a binding capacity similar to the wild type.

The selection results can also be used to draw conclusions about the number of improvements one can make by single amino acid mutations in this part of the enzyme. Despite the large number of substitutions of residues 433 to 439, only a few changes are accepted and selected for. That this number is indeed limited is also suggested by the finding that two of the mutants are independently selected for in two separate experiments at different pH value.

A closer look at the selected mutants indicates that selection of both single and double mutants has occurred, confirming the presence of both kind of mutants in the library. It has to be stressed, however, that only a fraction of the double mutants can be present in this library.

### Example 6: Properties of the selected mutants

The selected mutants have mutations in their starch binding domain. The integrity of the catalytic site is not directly affected. In principle the catalytic activity of the α-amylase and its binding to starch can be analysed separately by comparing the hydrolysis reaction on its natural substrate (starch), which requires binding, and its action to hydrolyse EPS, a small artificial substrate. The EPS is thought to require only binding of the maltoheptasoide in the active site, while for starch hydrolysis the enzyme has to adhere with its starch binding domain to the starch granule before the enzyme can scroll along the polymer and proper hydrolysis can occur.

The activity of the α-amylase on starch was be determined in two different ways: 1) by its action on azurin-crosslinked starch (Phadebas) which releases the blue coloured agent when the starch is degraded and by the hydrolysis of soluble starch followed by detection of the residual starch by iodine solution. The hydrolysis of the meltoheptasoide, which, in the presence of α-glucosidase yields nitrophenol, was studied in a continuous spectrophotometric assay.

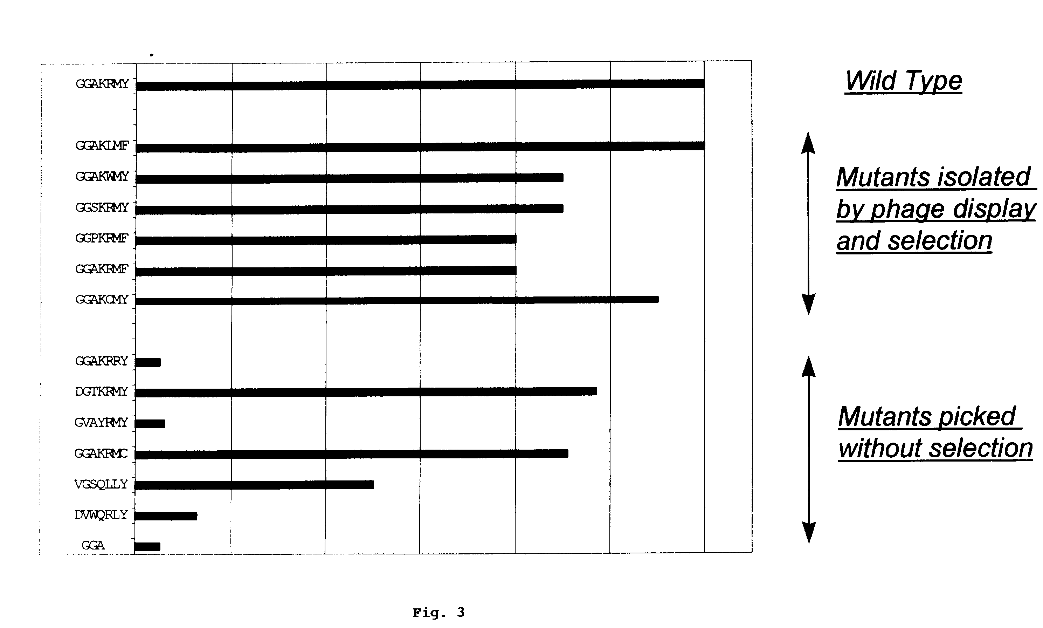

In all selected mutants the activity of the enzyme, both on starch as well as on the EPS was similar to the wild type activity. This means that the selected mutants have catalytic and binding properties which are similar to the wild type enzyme. The mutants that have been taken from the CB4 library without selection show a more divers pattern: some mutants have an almost wild type behaviour while other α-amylases have a decreased starch binding capacity. In all cases a decreased reaction on starch correlated with a decreased growth of the bacteria harbouring the mutant α-amylase. Figure 3 shows that the ratio of the activities on starch relative to the turnover on the EPS is indicative for the integrity of the starch binding domain. Mutants with a defective starch binding domain are expected to give a low ratio of activities in this test, unless their capacity to hydrolyse EPS is also affected.

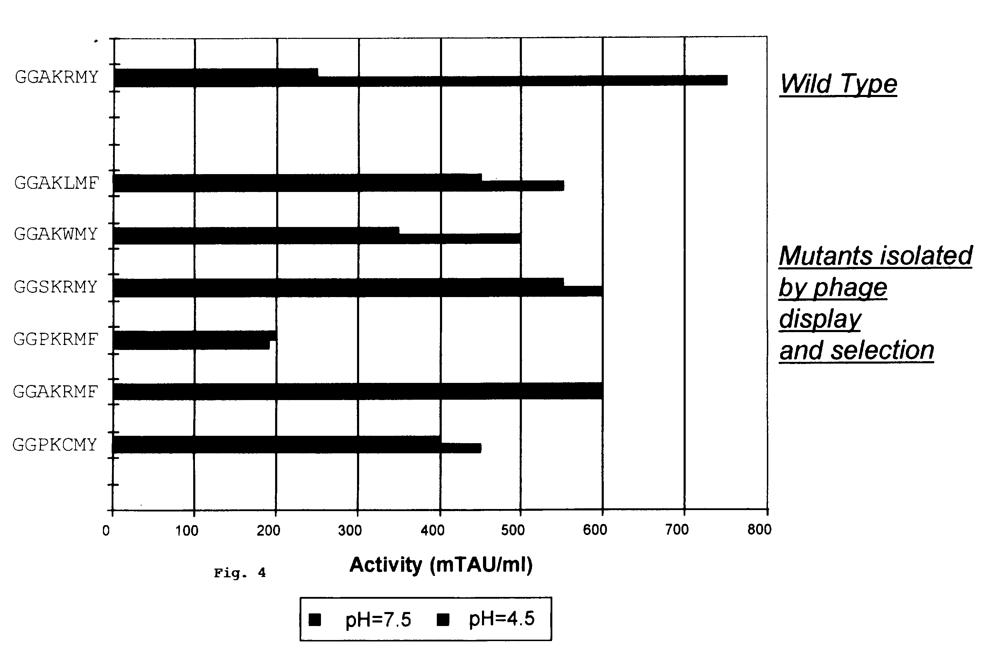

The results show that the our selection procedure yields mutants (and wild type) that have proper binding properties. This implies that we quickly can select a few but well equipped novel mutants out of the phage library using the one day quick select method variants. The activity measurements on starch were also determined at two different pH values: pH=7.5 and pH=4.5. In Fig. 4 the activity of the samples is presented. The ratio (pH=7.5:pH=4.5) of the wild type enzyme is approximately three, illustrating the decrease of activity of the wild type enzyme when the pH is lowered. The same measurements were performed on mutant isolates. In this case the ratio between the activity on pH=4.5 and pH=7.5 is closer to one. This indicates that the decrease in pH does not affect the selected mutants as markedly as it does the wild type. Therefore we can conclude that the activity of the selected amylase at lowered pH=4.5 is improved in comparison to the wild type.

### LEGENDS TO THE FIGURES

Fig. 1: Plasmids used for display and selection of α-amylase. Layout of the plasmid derived from pCANTAB 5E and pPGA-H-g3p

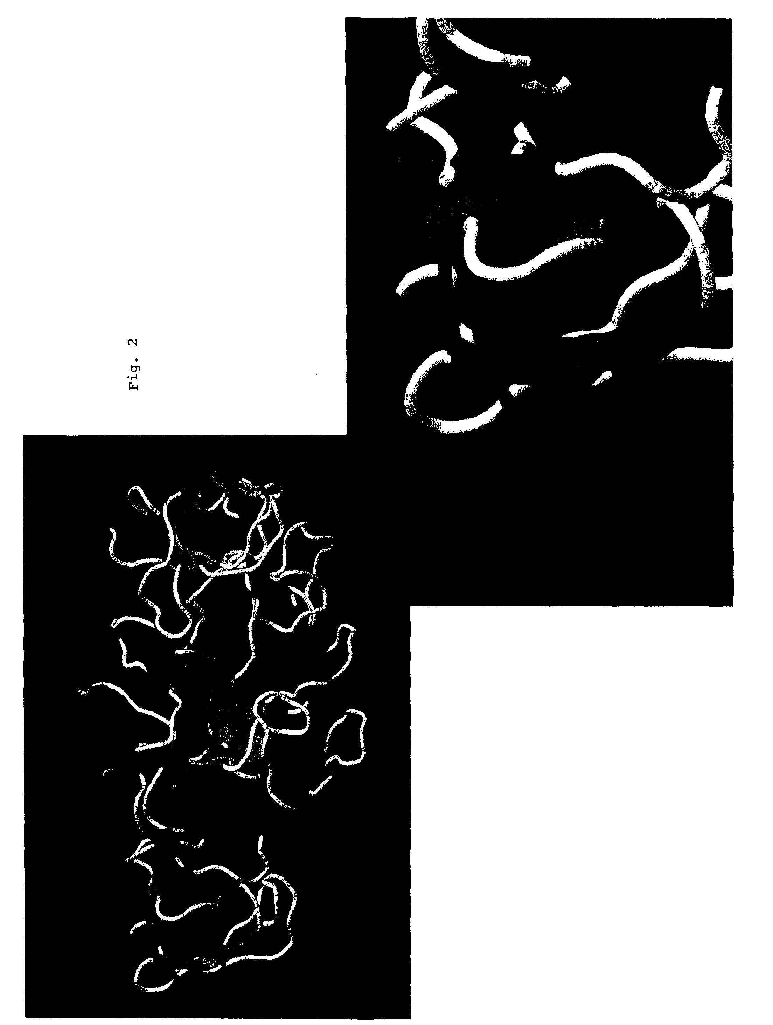

Fig. 2: Region of α-amylase used for selection.
   A. General structure of the α-amylase. The active site residues are represented in blue, the residues of CB4, which were randomized in the phage library are represented in green.
   B. Detail of the starch binding domain of α-amylase. Colour pattern as in A.
Fig. 3: Activity of α-amylase on starch in relationship to activity on heptamaltose. The relative activity of periplasmic proteins from E.coli cells containing mutant α-amylase on maltoheptose, requiring only active site activity, to the activity on starch, requiring both starch binding and hydrolysis.
Fig. 4: Activity of selected mutants at different pH values. The activity on starch of mutant and wild type α-amylase in E.coli periplasm at pH=7.5 to pH=7.5.

### LITERATURE

Cortese R, Felici F, Galfre G, Luzzago A, Monaci P, Nicosia A: Epitope discovery using peptide libraries displayed on phage. *Trends Biotechnol* (1994)**12**:262-267.

Crabb WD, Mitchinson C: Enzymes involved in the processing of starch to sugars. *Trends in Biotechnology* (1997)**15**:349-352. Helbert W, Schulein M, Henrissat B: Electron microscopic investigation of the diffusion of Bacillus licheniformis α-amylase into corn starch granules. *International Journal of Biological Macromolecules* (1996)**19**:165-169.

Hwang KY, Song HK, Chang C, Lee J, Lee SY, Kim KK, Choe S, Sweet RM, Suh SW: Crystal structure of thermostable α-amylase from Bacillus licheniformis refined at 1.7 A resolution. *Mol Cells* (1997)**7**:251-258.

Machius M, Wiegand G, Huber R: Crystal structure of calcium-depleted Bacillus licheniformis α-amylase at 2.2 A resolution. *Journal of Molecular Biology* (1995)**246**:545-559. Miller JH, Ganem D, Lu P, Schmitz A: Genetic studies of the lac repressor. I. Correlation of mutational sites with specific amino acid residues: construction of a colinear gene-protein map. *J Mol Biol* (1977)**109**:275-298.

Model P., Russel, M.: Filamentous Bacteriophage. In *The Bacteriophages (vol. 2),* 1st edn. edited by Calendar R. New York: Plenum Press; 1988:375-456.

Penninga D, van der Veen BA, Knegtel RM, van Hijum SA, W. Quax, Phage-enzymes: chances and limits, IBC's third Annual International Conference on Display Technology. Held June 11-12, 1998, The Clift House, San Francisco, USA.

Rozeboom HJ, Kalk KH, Dijkstra BW, Dijkhuizen L: The raw starch binding domain of cyclodextrin glycosyltransferase from Bacillus circulans strain 251. *J Biol Chem* (1996)**271**:32777-32784.

Roberts BL, Markland W, Siranosian K, Saxena MJ, Guterman SK, Ladner RC: Protease inhibitor display M13 phage: selection of high-affinity neutrophil elastase inhibitors. *Gene* (1992)**121**:9-15.

Rozie H, Somers W, van't Riet K, Rombouts FM & Visser J (1991) *Carbohydrate polymers* **15**, 349-365.

Satoh E, Niimura Y, Uchimura T, Kozaki M & Komagata K (1993) *Appl. Environm. Microbiol.* **59**, 3669-3673.

Smith GP: Filamentous fusion phage: novel expression vectors that display cloned antigens on the virion surface. *Science* (1985)**228**:1315-1317.

Somers W, Koenen P, Rozie H, Visser J, Rombouts F & van't Riet K (1995) *Enzyme Microb. Technol.* **17**, 56-62.

Soumillion P, Jespers L, Bouchet M, Marchand Brynaert J, Winter G, Fastrez J: Selection of beta-lactamase on filamentous bacteriophage by catalytic activity. *J Mol Biol* (1994)**237**:415-422.

Stanssens-P; Opsomer-C; McKeown-YM; Kramer-W; Zabeau-M; Fritz-HJ (1989) *Nucl. Acids Res.* **17**, 4441-4454.

Weber M, Foglietti MJ & Percheron F (1976) *Biochimie* **58**, 1299-1302.

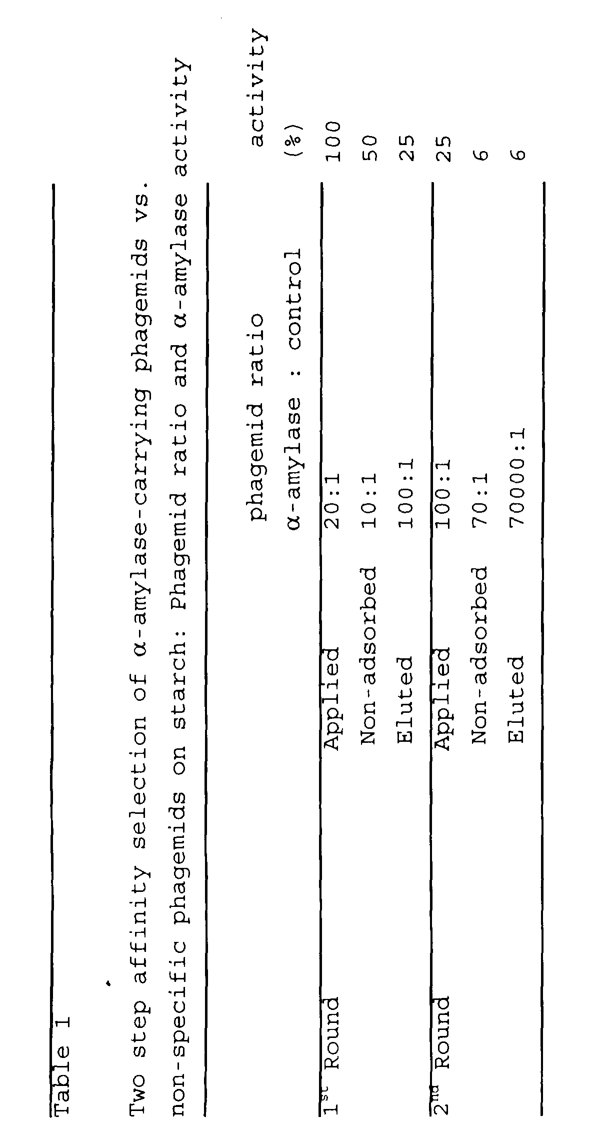

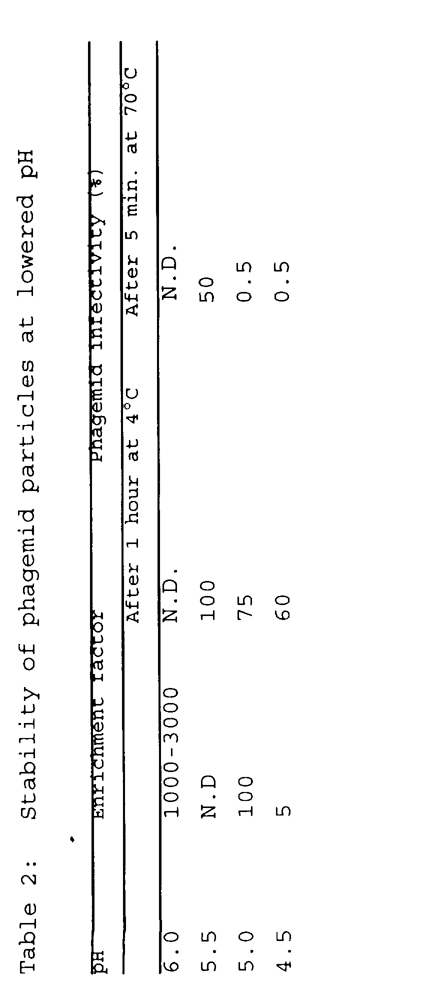

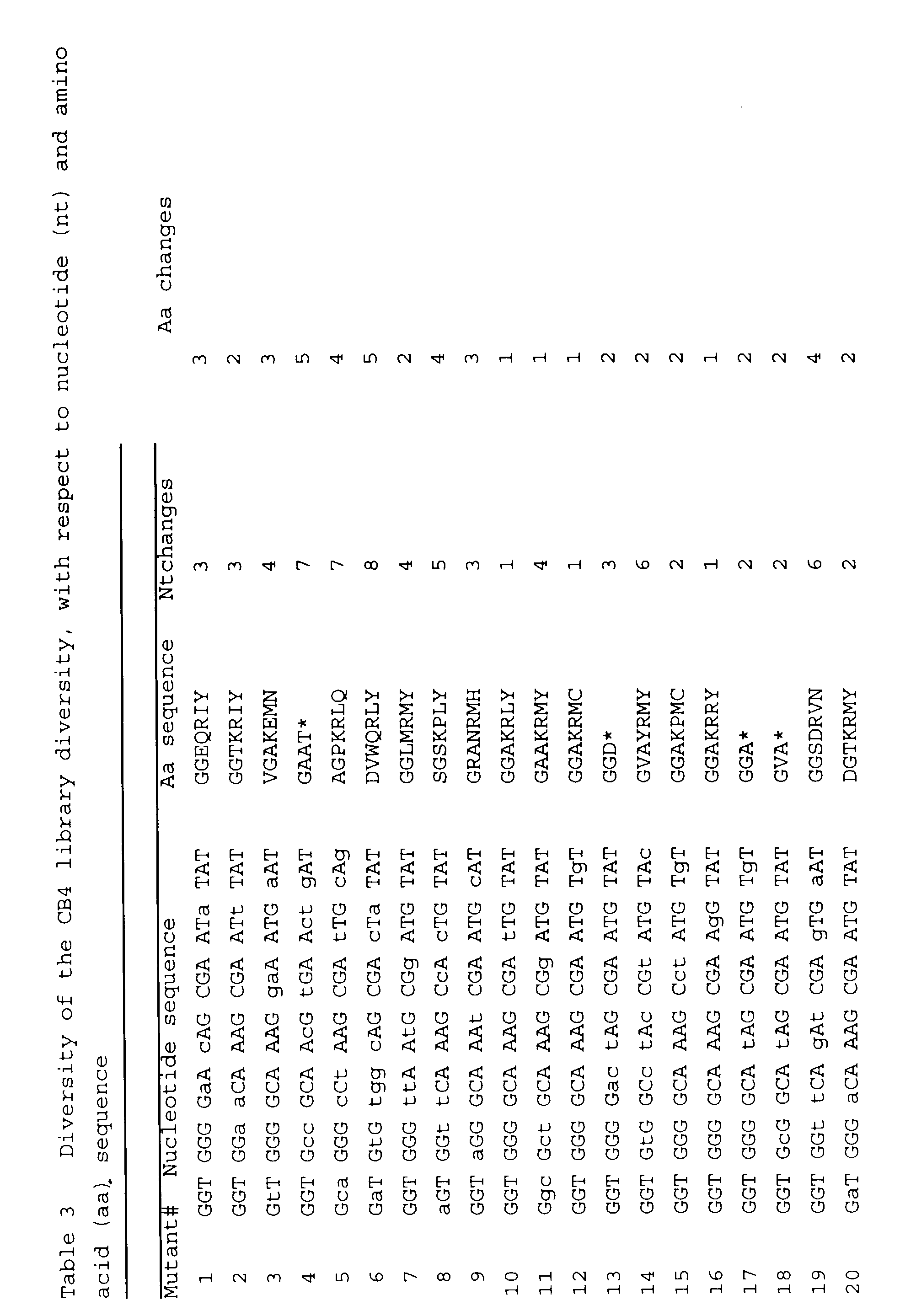

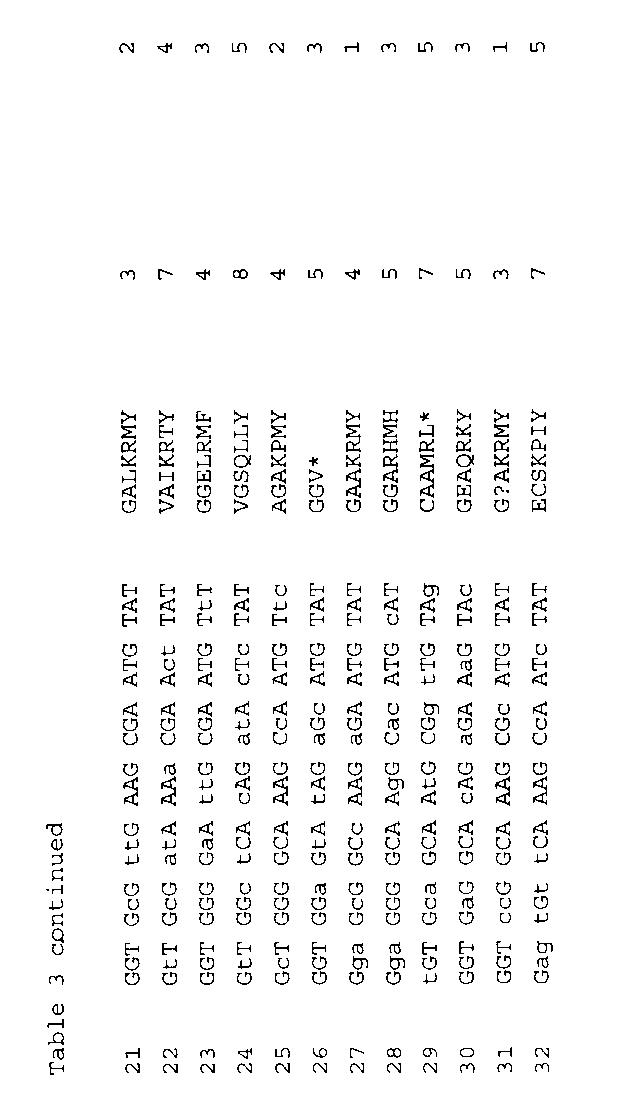

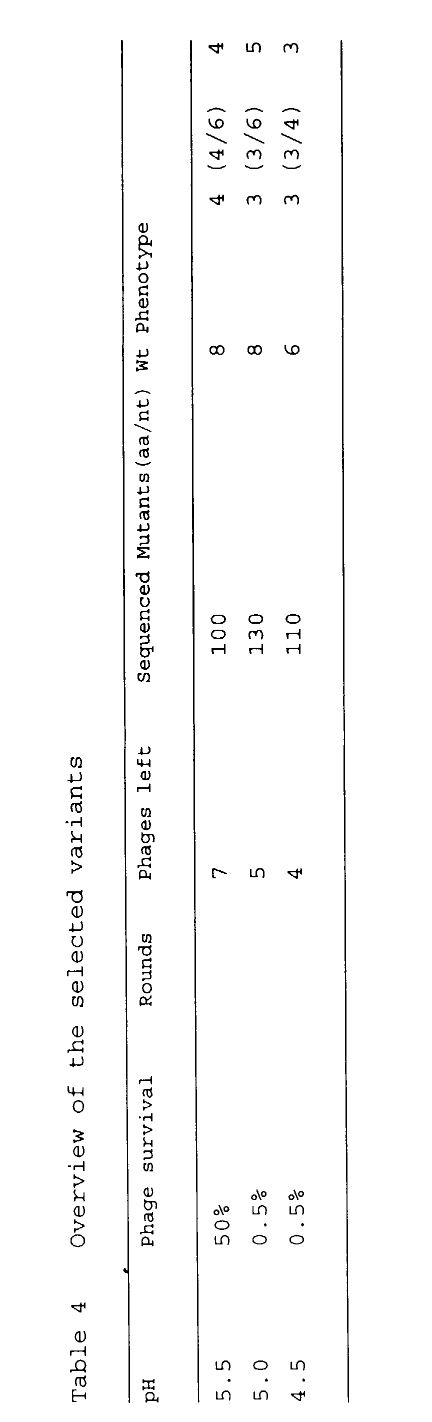

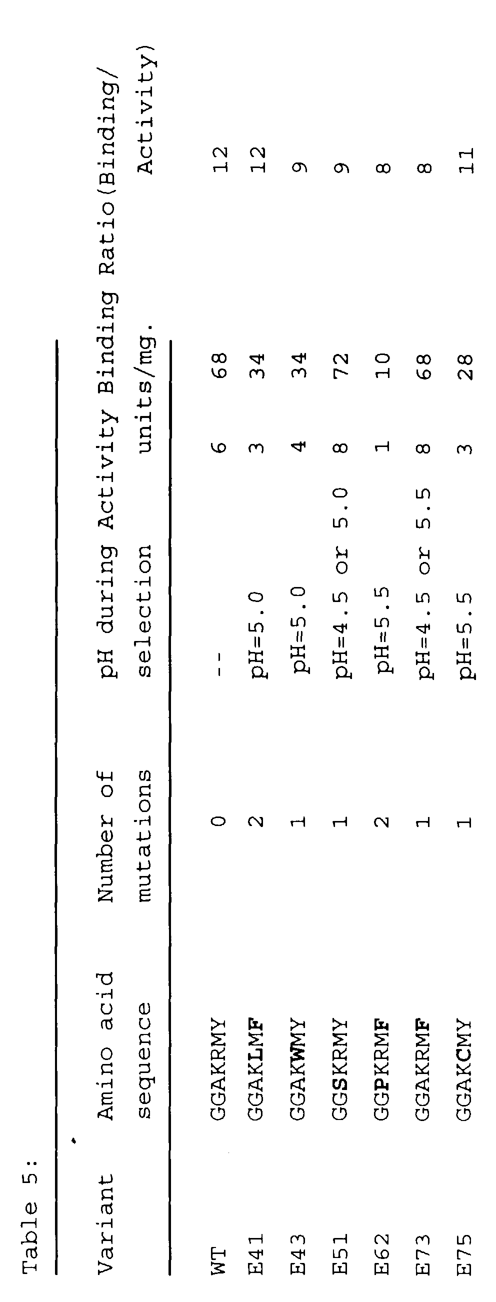

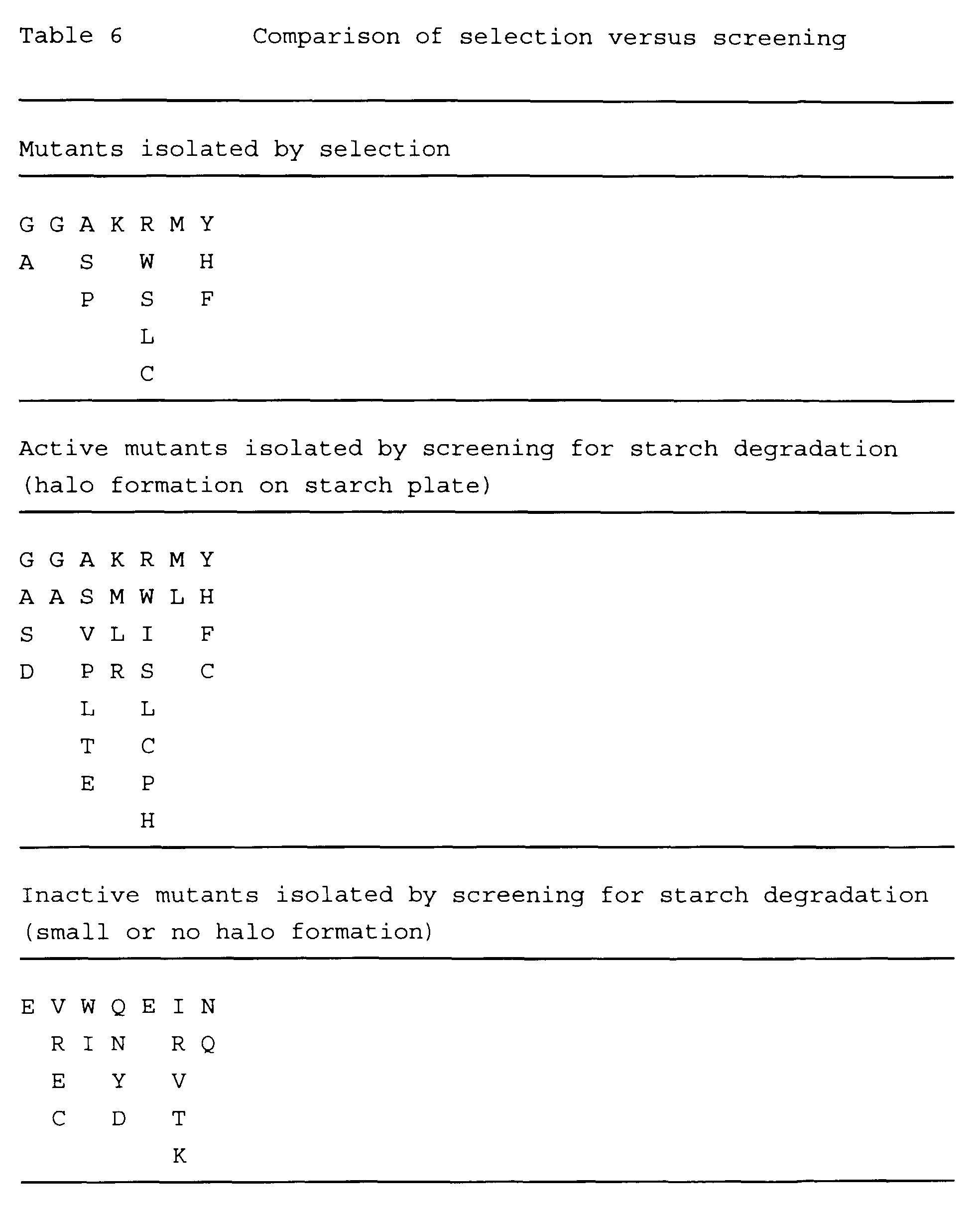

## Claims

1. A method for selecting an enzyme mutant with desired catalytic activity comprising displaying each of a multitude of mutants of said enzyme on a surface of a display vehicle comprising a nucleic acid encoding said mutant and selecting for a display vehicle carrying nucleic acid encoding an enzyme mutant having at least a site other than its catalytic site mutated.

2. A method according to claim 1 wherein said mutated site comprises a substrate binding site or allosteric site of said enzyme.

3. A method according to claim 1 or 2 further comprising selecting for improved binding of said enzyme displayed on said surface with a substrate for said enzyme.

4. A method according to claim 1 or 2 further comprising selecting for improved binding of said enzyme displayed on said surface with an effector for said enzyme.

5. A method according to claim 3 or 4 wherein said binding occurs under a set environmental condition.

6. A method according to claim 5 wherein said environmental condition comprises a condition capable of influencing said binding and/or said catalytic activity, such as presence or concentration of electrolyte, salt, substrate or product, heterologous enzyme or effector, or temperature.

7. A method according to claim 5 or 6 wherein said set environmental condition comprises a set pH.

8. A method according to anyone of the above claims wherein said binding of said enzyme displayed on said surface is tested in at least two rounds of selection.

9. A method according to claim 8 wherein at least between two rounds of selection said display vehicle is not amplified.

10. A mutant enzyme obtainable or selectable according to anyone of claims 1 to 9.

11. A mutant enzyme according to claim 10 having a catalytic site that is at least partly distinct from its substrate binding site or its allosteric site.

12. A mutant enzyme according to claim 10 or 11 being a polysaccharide binding enzyme.

13. A mutant enzyme according to anyone of claims 10 to 12 having improved catalytic activity over its wild-type enzyme at a set environmental condition.

14. A mutant enzyme according to claim 13 wherein said environmental condition is the pH value under which catalytic activity is determined.

15. A mutant enzyme according to anyone of claims 10 to 14 being α-amylase.

16. A mutant enzyme according to claim 15 derived from *Bacillus licheniformis.*

17. A mutant enzyme according to claim 15 or 16 having improved catalytic activity over its wild-type enzyme at pH 4.5.

18. A nucleic acid encoding a mutant enzyme according to anyone of claims 10 to 17.

19. A vector comprising a nucleic acid according to claim 18.

20. A host cell comprising a nucleic acid according to claim 18 or a vector according to claim 19.

21. A method to obtain a desired product comprising altering a substrate by subjecting said substrate to the catalytic activity of an enzyme according to anyone of claims 10 to 17.

22. A method according to claim 21 wherein said substrate comprises a polysaccharide.

23. A product obtainable by a method according to claims 21 or 22.

24. A method for selecting a mutant protein or mutant part thereof comprising displaying each of a multitude of mutants of said protein or part thereof on a surface of a display vehicle comprising a nucleic acid encoding said mutant and selecting for a display vehicle carrying nucleic acid encoding a mutant by successive rounds of selection wherein at least between two successive rounds of selection said display vehicle is not amplified.

25. A mutant protein or mutant part thereof selectable or obtainable according to a method according to claim 24.

26. A nucleic acid encoding a protein or part thereof according to claim 25.

27. A vector comprising a nucleic acid according to claim 26.

28. A host cell comprising a nucleic acid according to claim 26 or a vector according to claim 27.
